(19) Europäisches Patentamt
European Patent Office
Office européen des brevets

(11) EP 0 469 772 B1

(12) **EUROPEAN PATENT SPECIFICATION**

(45) Date of publication and mention
of the grant of the patent:
**15.10.1997 Bulletin 1997/42**

(51) Int Cl.6: **G01N 21/78**, G01N 17/00,
G01N 33/18

(21) Application number: **91306683.3**

(22) Date of filing: **23.07.1991**

(54) **Analysis of ferrous ion in circulating water**

Nachweis des Eisen(II)ions in strömendem Wasser

Analyse de l'ion ferreux dans de l'eau en circulation

(84) Designated Contracting States:
**AT DE ES FR GB IT NL**

(30) Priority: **31.07.1990 US 560680**

(43) Date of publication of application:
**05.02.1992 Bulletin 1992/06**

(73) Proprietor: **NALCO CHEMICAL COMPANY
Naperville Illinois 60563-1198 (US)**

(72) Inventors:
• **Pierce, Claudia C.
Lisle, Illinois 60532 (US)**
• **Fowee, Roger W.
Wheaton, Illinois 60187 (US)**

• **Banks, Rodney H.
Naperville, Illinois 60563 (GB)**

(74) Representative: **Harrison, David Christopher et al
MEWBURN ELLIS
York House
23 Kingsway
London WC2B 6HP (GB)**

(56) References cited:
DE-A- 3 221 063          GB-A- 894 459
GB-A- 1 582 228          US-A- 3 874 794

• **PATENT ABSTRACTS OF JAPAN vol. 7, no. 241
(P-232)(1386) 26 October 1983 & JP-A-58 127 152
(KIYUUSHIYUU DENRIYOKU K.K.) 28 July 1983**

## Description

This invention relates to analyzing ferrous ion in a steam generating system.

Problems resulting from boiler corrosion seriously affect the normal operation and efficiency of steam-generating equipment. Most often it is the deposition of particulate iron oxides from the condensate system that is the major cause of tube failure and efficiency loss. In the condensate system, iron corrosion is facilitated by dissolved oxygen and carbon dioxide, viz.,

$$Fe + 2H_2CO_3 \rightarrow Fe^{2+} + 2HCO_3^- + H_2\uparrow$$

$$4Fe + 6H_2O + 3O_2 \rightarrow 4Fe(OH)_3$$

High temperature and stresses in the boiler accelerate the above reactions where carbonic acid is the result of dissolved $CO_2$. Low feedwater pH and high chelate residuals can also produce iron corrosion.

Despite chemical treatment progress and mechanical deaeration equipment, corrosion is often quite active in an operating boiler. The ability to monitor corrosion and minimize its effects is necessary for efficient long-term operation.

As steel begins to corrode, $Fe^{2+}$ (hereinafter Fe(II)) is formed ("new iron") and remains as a soluble species for some time in the nearly oxygen-free environment of a boiler. Ferric ion, $Fe^{3+}$ (hereinafter Fe(III)) is termed "old iron" and is generally insoluble and can originate from several sources including feedwater. By monitoring selectively for Fe (II), information on the extent of active corrosion relative to an earlier time period can be obtained. Unlike the case of Fe(III), soluble Fe(II) is not subject to particulate sampling errors. However, one must be sure to exclude air contact as Fe(II) is readily oxidized to Fe(III).

Since corrosion is a dynamic and continuous process that depends on boiler conditions and chemical treatments, it would be advantageous to monitor the corrosion process on-line, and one object of the invention is to accomplish this.

It has been found that one of the most sensitive reagents for the determination of low levels of Fe is 3-(2-pyridyl)-5,6-bis-(4-phenyl sulfonic acid)-1,2,4-triazine monosodium monohydrate, for example FerroZine (Registered Trade Mark), supplied by Hach Chemical Company. The chemistry involved is disclosed in our EP application filed on the same day as the present and entitled "Iron (II) Concentration Monitoring for Determining Corrosion in Boiler Systems".

Another object of the invention is to take advantage of this in determining Fe(II) concentration. This chemical will be abbreviated FZ. The ferrous ion/FZ complex has a large molar absorptivity of $2.86 \times 10^4$ mol$^{-1}$-cm$^{-1}$ at 562 nm at pH 3-6. Ferrozine itself and its Fe(III) complex exhibit negligible absorbance at this wavelength. The color-forming reaction is rapid. All reactants and products are water soluble and have no adverse effect on the industrial or municipal water systems involved. Thus, the only reagent needed for the analyzer is an aqueous solution of FZ containing an acetate buffer near pH 3.6. Additional optional reagent components are a fluorosurfactant and inert electrolyte. The components and their concentrations for the reagent will be given below. The ratio of reagent to sample is preferably 1:50 by volume.

The concentration of soluble Fe(II) in an aqueous sample is quantified by reacting it with FZ, resulting in a ferrous ion/FZ color complex in which the intensity is proportional to Fe(II) concentration. The absorbance of the sample is measured as a proportional analog of the concentration of the iron. This absorbance analog is converted to a voltage, enabling Fe(II) values to be read and acted upon. In particular, variations in the ratios of new iron to old iron may be calculated as a cross-section of performance history or trend.

In the drawings:

Fig. 1 is a diagram of a steam generating boiler system;
Fig. 2 is a diagram of the analyzer of the present invention;
Fig. 3 shows calibration factors for the analyzer; and
Fig. 4 is a block diagram of the analyzer presenting more detail compared to Fig. 2.

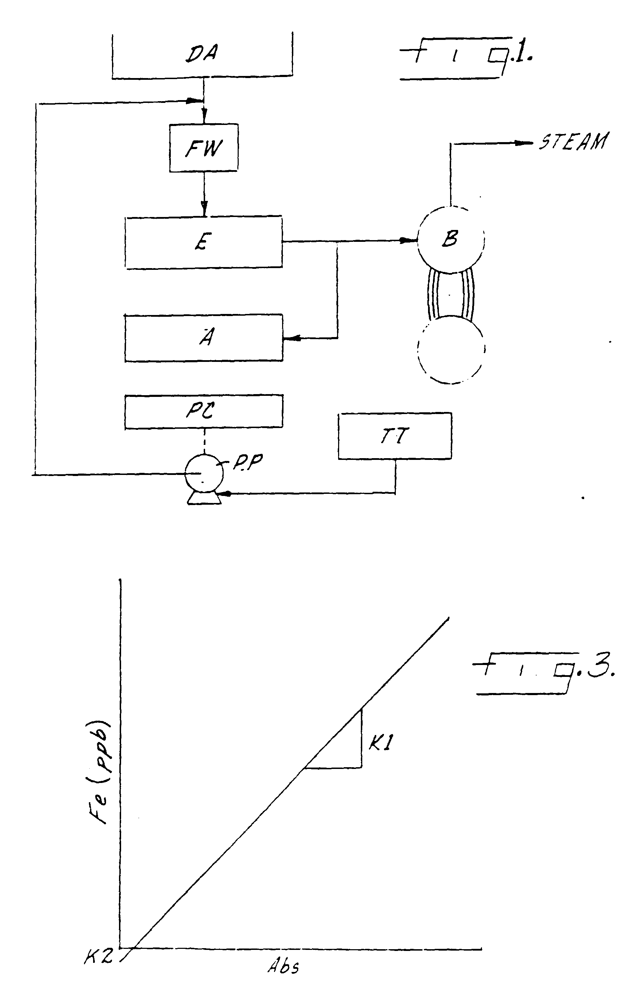

A typical steam generating system is shown in Fig. 1. Make-up water MW and condensate return CR are fed to the deaerator DA by a feed water pump FW and passed to the economizer E which feeds the boiler B.

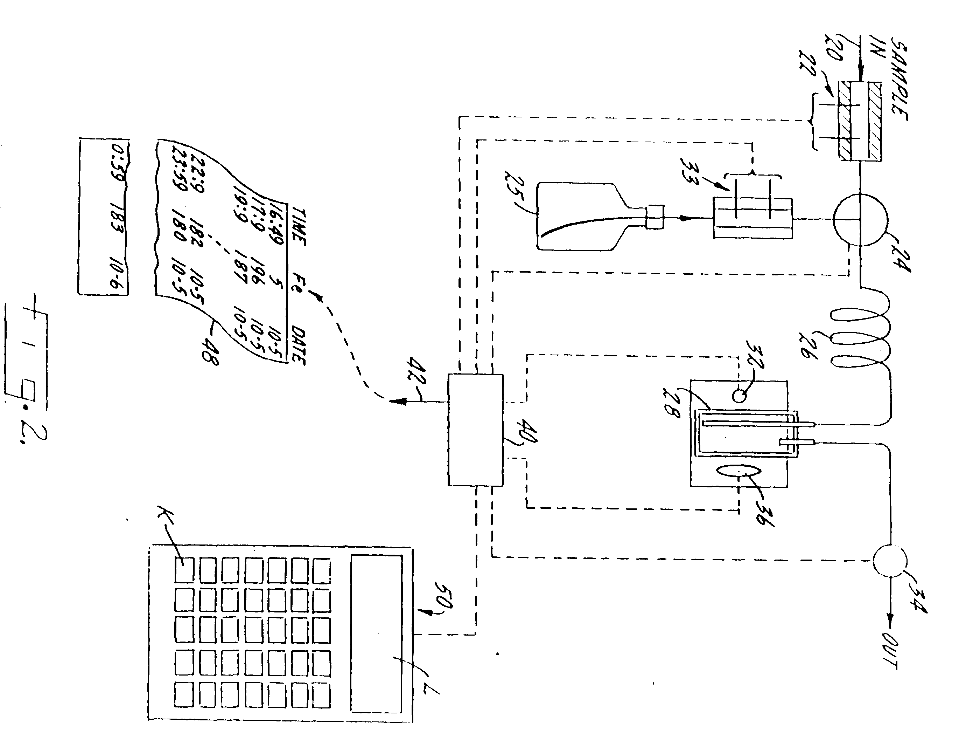

In accordance with the present invention, the analyzer A for ferrous ion' diagrammed in Fig. 2 in general terms, may be used to sample water going from the economizer to the boiler B.

A source or supply of corrosion inhibitor TT (chemical) is ordinarily connected to a pump PP used (manual control) to dose the system water with a known chemical from source TT. For example, pump PP may control dosage of inhibitor to the water being passed from the deaerator to the economizer.

The sample to be analyzed for ferrous ion may be taken almost anywhere in the steam system known to be susceptible to corrosion due to dissolved oxygen and carbon dioxide. These areas would include the preboiler (steam drum, mud drum, riser and downcomer tubes) and the condensate systems. Regardless, reactions of the iron surfaces with $O_2$ or $CO_2$ result in attack on the steel equipment, characterized by release of ferrous iron, and it is this new iron which in to be calculated under and in accordance with the present invention. At all times, the sample to be calculated for Fe(II) concentration in air-free to avoid oxidation of Fe(II) to Fe(III).

The method and apparatus are generally described with reference to Fig. 2. The sample is passed through a flexible conduit 20 and its presence (sample present) is detected by a sensor 22 which has platinum electrodes.

The sample is passed to a three-way valve 24 where FZ reagent from a source 25 is injected, from there through a delay or mixing coil 26 and from the mixing coil to the analyzer cell 28 illuminated by an LED 32.

A sensor 33 is preferably employed to sense near depletion of the reagent in bottle 25.

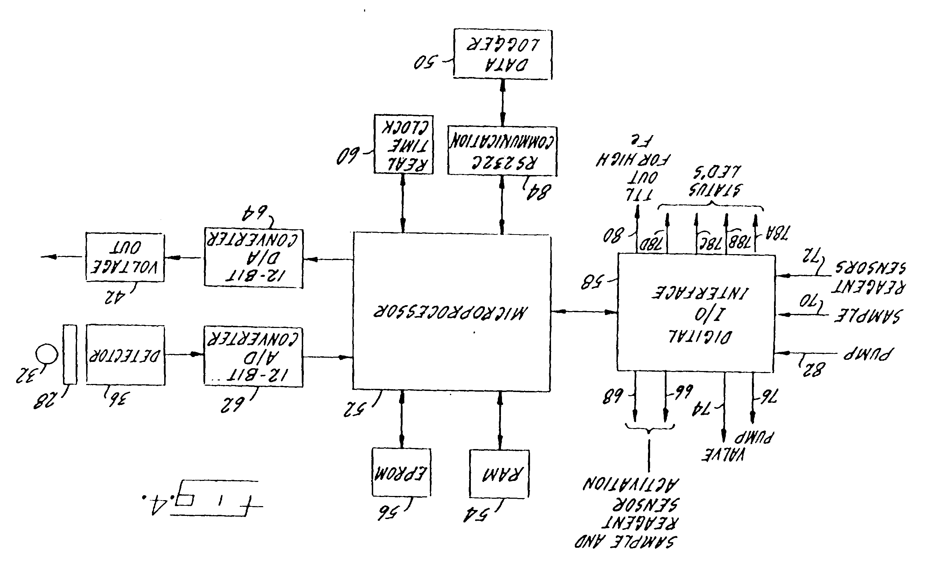

The sample is drawn or forced to the cell by a synchronous pump 34 which returns the sample to waste. The pump is momentarily disabled while the sample is in the cell to be sure the peak absorbence value is reached, absorbance being registered by a detector 36 (photodiode) which converts the transmitted light to a voltage analog which is proportional to the ferrous concentration. This voltage value is transmitted to the electronic control circuit 40. It will be noted from Fig. 4 that a microprocessor in the electronic control circuit 40 also receives information concerning sample presence, reagent supply, three-way valve position, and pump status among other data.

The control circuit 40 has a voltage output in the form of a recording output 42. The output 42 may be employed to print a continuing log 48 of time/Fe concentration/date for example.

A pump PP may be started by a pump controller PC to add inhibitor, or the pump may be stopped to discontinue dosing the system, or it may be held at a steady state to hold Fe(II) at an acceptable value.

The keys K of a hand-held terminal or datalogger 50 can be used to sequence the microprocessor as desired; data are displayed on its panel L.

In Fig. 2 the sections through which the sample is moved and injected with the reagent may be termed the "manifold."

Reagent Composition

The reagent is the Ferrozine reagent composition (FZ) in which all materials are dissolved in distilled or deionized water. Thus, for 1 liter of reagent in the reagent bottle 25:

100 gms sodium acetate trihydrate
75 gms glacial acetic acid
3.0 gms Ferrozine powder
2.0 gms Zonyl FSN fluorosurfactant
0.2 gms high purity KC1

System Conditions

The system conditions for accurate analysis of the sample are broad. The ambient temperature may be 5-50°C.; humidity non-condensing at 0-95%. For the sample itself:

| | |
|---|---|
| Temperature | 10-40°C. |
| Pressure | 0-5 psig |
| Prefilter | ~100 microns |
| Minimum flow rate available | - 10 mls/min |
| Soluble Fe(II) concentration range | - 2-1000 ppb |

Instrumentation Specifications

The following operating parameters or limit specifications for the instrumentation are as follows (where "1" symbolizes software changes may be made and "2" includes manifold purge time):

| | |
|---|---|
| Fe(II) measurement range[1] | 2-1000 ppb |
| Display resolution[1] | 1 ppb |
| Reproducibility | 3% |
| Absolute accuracy | 5% |
| Analysis time [1,2] | 3 minutes (approx) |
| Reagent: Sample ratio | 1:50 by volume |
| Sample volume per analysis[1] | 42 mls |
| Reagent bottle volume | 500 mls |
| Reagent supply interval[1] | 30 days |
| Frequency of Operations[1] | |
| Analysis | 6 per hour |
| Calibration | manual |
| Operating modes | Automatic analysis with auto restart upon power up reset[1] Calibration User invoked standby Manual operation of pump and valve |
| User interface | 16x2 line alphanumeric, scrolling |
| Input-Output | ASCII terminal, RS232C |
| Status lights | Purge, Test, Wait, Error |
| Signal outputs[1] | 0-10 Vdc, nonisolated |
| Diagnostics[1] | Error messages for: pump failure, out of reagent, loss of sample, no calib factors |
| Security | Operating program cannot be permanently changed by operator |
| Sample inlet/outlet | 1/8" nylon Swagelok |
| Input power | 110VAC, 50/60 Hz, 100 W (max) |

Calibration, Fig. 3

The equipment is preferably calibrated between samplings, or from time to time. In this regard, references may be made to Fig. 3 in which the concentration of the ferrous ion is shown as a function of absorbance. More specifically,

$$K1 = H/[Abs(\text{High std})-Abs(\text{zero std})]$$

$$K2 = -K1*Abs(\text{zero std})$$

$$Fe = K1*Abs(\text{sample})+K2$$

A negative K (-K) features because if Fe(II) concentration is less than 5 ppb there is a slight change in its refractive index, needing compensation.

In the above equation "High std" (also H) is chosen to represent the highest Fe(II) concentration standard of interest; "zero std" contains no Fe(II). Thus, if the high standard is 500 ppb having an absorbance equivalent of 0.2, and if the absorbance equivalent for the zero standard is 0.002, then

$$K1 = 500/0.2 - 0.002$$

$$K2 = -K1 (0.002)$$

which are values retained in nonvolatile RAM along with the equation for determining the Fe(II) concentration of the sample, namely,

$$Fe(II) = K1Abs(\text{sample}) + K_2$$

Calibration can be performed by pressing the calibration button on the analyzer, which starts a menu-driven, two-step process. The first standard run is a zero standard containing no Fe(II). The second standard is chosen to represent the highest Fe(II) concentration of interest, i.e., "High Std". Calibration factors K1 and K2 are calculated from the four absorbance readings, displayed and stored until another calibration is run.

Detailed Description of the Manifold

The reagent bottle is preferably a clear, graduated 500 ml polycarbonate bottle having a plastic cap fitted with an 1/8" O-seal male Swagelok connector, drilled through to allow a piece of Teflon tubing to extend to the bottom of the bottle. The other end is connected to the NC port of the solenoid valve. Two small holes are drilled into the neck of the fitting to permit air to enter as reagent is withdrawn while minimizing evaporation.

Valve 24 is a 110 Vdc Teflon three-way solenoid valve (Neptune Research Maplewood, NJ). It has a common outlet with NO and NC ports for sample and reagent lines, respectively.

The flow cell 28 is a quartz flow cell having 1 cm path length and 3 mm dia aperture (80 micro liters volume; Hellma Cells, Jamaica, NY). For ease of connection to the manifold, it has special screw fittings for 3/32" dia Teflon tubing. A cell holder (not shown) may be machined of aluminum to securely hold the cell, LED source, detector, and their circuit boards. The whole assembly may be housed in a grounded aluminum box.

A preferred form of synchronous pump 34 is that of Fluid Metering Inc., Oyster Bay, NY, model RHOCKC pump head with ceramic piston and Kynar body. It achieves excellent 1% precision by use of a 110 VAC synchronous motor. Maximum volume per stroke is .05 ml at selectable stroke rates of 150, 300, 600 strokes per minute; the lowest stroke rate was chosen. A Hall effect sensor is preferably included to monitor proper operation of the synchronous pump. While the pump is shown at the end of the manifold, it could also be placed between the mixing coil 26 and flow cell 28 to reduce the tendency of bubble formation in the cell.

Sensors 22 and 33 for the sample and reagent are constructed from 1/2" diameter Teflon bar stock. A 1/8" diameter hole is drilled through the axis of a 1 1/2" long piece and the ends were tapped to accommodate Teflon tubing fittings. Two .5 mm diameter platinum wires can then be press fit through the body so that they will protrude into the 1/8" hole. One of the Pt electrodes is permanently grounded while the other is connected to a voltage source and a sensing line. Said other electrode makes contact to ground via liquid in the sensor, if liquid is present.

The entire manifold is constructed of components of high mechanical reliability and noncontaminating surfaces. Teflon tubing (1/16"ID, 1/8"OD) is used for the mixing coil 26 and to transport sample and reagent throughout the system. The sample and reagent do not contact any metal (except for the two Pt electrodes in the sample and reagent sensors). Nylon tubing connectors having Teflon ferrules are employed to ensure leak-free and reusable connections.

The volume of the sample manifold is 5 ml so that the proper amount of reagent can be added with exactly two pump cycles. Since the pump output is .05 ml per stroke, two pump cycles will add .10 ml of reagent. Given the 5 ml volume of the manifold, the desired reagent/sample ratio of 1:50 is obtained with two pump cycles.

Block Diagram of Electronics - Fig. 4

The electronic control circuit 40 is shown in block diagram form in Fig. 4. An eight-bit microprocessor 52, such as an Intel 8052AH-Basic, is used for controlling all the operations required. External components including an address latch, address decoder, crystal, and serial port driver are added to the microprocessor. These components are standard and are not shown in Fig. 4.

The microprocessor 52 employs a 32K RAM shown at 54. The controller software is stored in a 16K EPROM shown at 56. The 8052 processor has 16 address lines enabling it to span 64K of external devices. The RAM 54 and EPROM 56 take up 48K of address space. The remaining 16K are devoted to a programmable peripheral interface (PPI) 58, real time clock/calendar 60, a 12-bit A/D converter 62, and a 12-bit D/A converter 64. A brief description of the major components follows.

The PPI is the main interface between the CPU and the outside world. It may be an Intel 8255A. It has three 8-bit parallel I/O ports. Twelve of the 24 bits are used for the following functions: a) to activate the digital sensors 22, 33 for the sample and reagent through lines 66 and 68, respectively. This tests for the presence of sample and reagent in the manifold; b) to read the signals from the sample and reagent sensors on lines 70 and 72; c) to control the valve 24 on line 74, normally setting it to permit the sample to flow into the manifold but periodically setting it to open the reagent flow line for two cycles of pump 34; d) to control pump 34 on line 76, shutting it off periodically to allow quiescence in the cell 28; e) to control four LED's on lines 78A-D. The LED's provide visual indication of system status, such as wait, test, purge and error; and f) to provide TTL logic output on line 80 for indicating an Fe content beyond the calibration range.

The PPI 58 also has a line 82 for reading whether the pump 34 is operating in the proper frequency range. The Hall effect sensor on the pump generates a low pulse for each rotation or pump cycle. At the setting chosen for the analyzer, the pulse frequency is 2.34 Hz. A phase-locked loop circuit (CD4046) having a $2^9$ divider (4040B) in the loop converts this to 1198 Hz. The resulting signal is AC coupled to a 567 tone decoder IC which then outputs a constant low output only if the input frequency is 1198 +/- 100 Hz. Otherwise a logic high is output. The 567 output is connected through an inverter to line 82 so proper pump operation can be verified.

Optically isolated solid state relays (SSR's, not shown) are employed to interface the TTL, outputs of the PPI and the 110V valve 24 and pump 34. This eliminates the possibility of 110V contacting any part of the logic system. The SSR's are rated at 1A which is more than adequate for the valve and pump which are rated at .01A and .07A, respectively.

Since the valve must be operated momentarily for exactly two complete pump cycles (850 ms), the SSR for it is activated by a 555 timer connected as a monostable multivibrator. Two switches may be provided to allow manual operation of the pump and valve when desired. While on the subject of valve operation, it is pointed out that the time of switching the valve 24 to the reagent bottle need not be synchronized to the beginning of a pump cycle. The valve need only be open for a time period of two pump cycles and it does not matter where in the pump stroke that time period starts and stops.

The clock/calendar 60 is used to determine the time and date that the Fe(II) value is taken. To ensure continued accuracy in a power off condition, battery backup is provided. This capability allows possible use of options such as automatic calibration every week, for example, or logging of important events such as power failures, errors, overrange Fe(II) values and reagent renewal warning.

The LED source 32 is a bright green LED of wavelength 565nm. It is powered by the main +5V logic supply at 20ma. Additional filtering is provided by a passive LCR circuit. The detector 36 is a Si photodiode/op amp module (HUV1100BG, by EG&G Solid State). It converts source intensity to voltage. Full scale output is set to 9V with approximately 12 M-ohm feedback resistance. The voltage is fed to a second-order Butterworth low pass filter, buffered, then to the input of the A/D converter 62.

Communication with the microprocessor is through a PSION terminal or datalogger 50 connected to an RS232C serial port driver 84. The terminal has a full function keyboard for typing in commands or to download data to another computer. In its capture mode, the terminal stores the date, time and Fe(II) data in its internal memory for later retrieval.

Operation of the Analyzer

Once the unit is calibrated, the microprocessor is programmed to operate as follows. The analysis is started by turning the pump on to thoroughly flush the manifold with fresh sample. At 20 seconds into the purge operation, the unit checks for proper pump operation and whether sample and reagent are present. If any of these are false, the unit goes into a standby mode, the error LED flashes, and an appropriate message is displayed on the terminal 50.

If the unit is operating properly, the purge is allowed to continue for another three and half minutes or so. Then the CPU signals the valve 24 to switch over, permitting reagent flow into the mixing coil 26. As described above, the valve is switched to allow reagent flow for a time period of two pump cycles, thereby drawing .10 ml Ferrozine reagent into the sample stream. There the reagent disperses into the 5 ml mixing coil. Just before the treated sample bolus reaches the flow cell 28, at about 18 seconds after reagent injection, the pump is shut off momentarily and a blank reading is taken of the pure sample. This provides a baseline value that reflects sample color and may taken at any time during the purge but it is preferred to take it as close in time as possible to the reading of the mixed sample and reagent. This is to minimize effects of detector drift. Immediately after the blank reading is taken, the pump is turned back on.

When the peak of the treated sample bolus fills the flow cell cavity, at about 20 seconds after the blank reading, the CPU shuts off the pump. The reaction is allowed to go to completion by maintaining a quiescent condition in the cell for 15 seconds. Then another reading is taken of the flow cell absorbance. This reading represents that of the ferrous ion/FZ complex. The CPU calculates the new Fe(II) value and displays it with the date and time. The pump is then reactivated to remove old sample. Once the old sample is gone, after about a minute and twenty seconds, the pump is turned off and the unit waits for about four minutes and twenty seconds to begin the next analysis.

To reduce random noise and increase accuracy of reading the voltage of detector 36, the software takes and averages 20 voltage readings for each absorbance measurement. The voltage output 42 from the analyzer spans 0 - 10 volts, where $0V = 0$ ppb Fe and $10V = $ [Fe] of the calibration standard used. Intermediate values are determined as a ratio of full scale. For example, if the Fe standard were 500 ppb, then the output voltage for a sample containing 200 ppb Fe would be 4V. The logic output signal on line 80 would be low for 0-500 ppb and high for [Fe] > 500 ppb.

It will be recognized the present apparatus may be used to monitor total iron in the sense the instrumentation herein disclosed analyzes Fe(II). Thus, a reducing agent may be employed to reduce Fe(III) to Fe(II) which then gives a measurement of total Fe(II) + Fe(III).

While one form of the invention has been shown and described, it will be understood that modifications could be made to the arrangement shown. For example, the flow cell could be angled at about 15 degrees to help prevent bubble entrapment. A check valve could be placed at the analyzer outlet to provide flow restriction and reduce bubble formation.

**Claims**

1. Apparatus for determining if corrosion, in terms of soluble Fe(II), prevails in water circulating in a steam generating system, comprising:

    a manifold including a flow cell (28);
    means (34) for withdrawing a sample of predetermined volume of the water to be tested without air contact and for circulating the sample through the manifold;
    means (24,25) for injecting into the sample a predetermined volume of reagent reactive with Fe(II) to produce a color intensity in the sample proportional to Fe(II) concentration;
    means (26) for mixing the combined sample and reagent in the manifold;
    means for controlling said means for withdrawing to stop the flow of sample for a first quiescent period prior to mixed sample and reagent reaching the flow cell, for thereafter restarting the flow of sample, for then stopping the flow for a second quiescent period after the mixed sample and reagent have reached the flow cell, and then restarting the flow to flush the manifold;
    means (32,36) for measuring the absorbance of the unmixed sample in the flow cell (28) during the first quiescent period and for measuring the absorbance of the mixed sample in the flow cell (28) during the second quiescent period; and
    means (40,42) for converting the measured absorbance values to Fe(II) concentration.

2. The apparatus according to claim 1 further comprising means (40) for outputting a voltage and TTL logic signal according to the value of the calculated Fe(II) concentration.

3. The apparatus according to claim 2 wherein that said means for outputting holds said outputs steady until the next analysis updates them.

4. The apparatus according to any one of the preceding claims wherein the controlling means includes means for stopping the sample flow in an idle mode to wait for the next analysis cycle.

5. The apparatus according to any one of the preceding claims wherein said means for withdrawing is a synchronous pump (34).

6. The apparatus according to claim 5 further comprising means for determining if the pump is operating at the proper frequency.

7. The apparatus according to any one of the preceding claims wherein the mixing means is a coil of tubing (26).

8. The apparatus according to any one of the preceding claims including means for converting the absorbance to a voltage analog from time to time and using that analog to produce a record of ferrous iron concentration over a like time span.

9. The apparatus according to any one of the preceding claims further comprising sensors (22) to sense passage of a sample to the cell (28) and to sense the supply of reagent.

10. The apparatus according to any one of the preceding claims further comprising a data logger and means for interfacing the data logger with said means for converting the measured absorbance values to Fe(II) concentration.

11. The apparatus according to any one of the preceding claims wherein the means for measuring includes a light source (32) of suitable wavelength and intensity.

12. The apparatus according to claim 11 wherein the means for measuring further comprises a detector (36) to transform the light source intensity to voltage.

13. The apparatus according to claim 12 wherein the flow cell (28) has transparent walls and the light source (32) and detector (36) are mounted adjacent said transparent walls such that the optical measurements can be made.

14. The apparatus according to any one of the preceding claims wherein the means for controlling starts the first quiescent period about 18 seconds after the means for injecting combines the reagent with the sample.

15. The apparatus according to claim 14 wherein the means for controlling starts the second quiescent period about 20 seconds after taking the measurement during the first quiescent period.

16. The apparatus according to claim 15 wherein the means for measuring reads the absorbance of the mixed sample about 15 seconds after the second quiescent period begins.

17. The apparatus according to claim 16 wherein the means for controlling allows the flush of the apparatus to last about one and a third minutes.

18. The apparatus of claim 1 further comprising a source (25) of reagent reactive with Fe(II) to produce a colour intensity proportional to Fe(II) concentration;

said means (32,36) for measuring the adsorbance of the unmixed sample in the flow cell (28) during the first quiescent period and for measuring the absorbance of the mixed sample in the flow cell (28) during the second quiescent period includes a light source and a photodetector;
and said means for converting the measured absorbance values to Fe(II) concentration includes means for converting the absorbance value to a voltage analogue proportional to Fe(II) concentration.

19. The apparatus of claim 18 wherein said means for withdrawing includes a conduit connected to a source of the water to be reacted with the reagent;

a valve having one inlet connected to the conduit, another inlet connected to the reagent source and an outlet connected to the mixing means;
means to switch the valve between one inlet or the other to allow either water or reagent to enter the mixing means; and
a pump to force water and reagent into the mixing means and from there into the flow cell (28).

20. The apparatus of claim 19 further comprising sensors for detecting the presence of water in the conduit or reagent in the reagent inlet to the valve.

21. The apparatus of claim 18 wherein the mixing means is a coil of tubing.

22. The apparatus of claim 18 wherein the means to convert comprises an analog-to-digital converter to convert the photodetector voltage to a digital signal, and a microprocessor programmed to calculate the Fe(II) concentration from said digital signal.

23. The apparatus of claim 1 further comprising a source of a reagent reactive with Fe(II) to produce a colour intensity proportional to Fe(II) concentration,

> said means for withdrawing including a conduit, a valve to allow a predetermined volume of the reagent from a source to be combined with the sample and means to open and close the valve
> said means for controlling said means for withdrawing including a pump to force the reagent-combined sample into the cell
> said apparatus further comprising timing means to stop the pump for a predetermined time while the sample is measured for absorbance
> and means for converting the absorbance value to a voltage analog proportional to Fe(II) concentration

## Patentansprüche

1. Vorrichtung zur Bestimmung, ob Korrosion - in Form von löslichem Eisen(II) - in Wasser vorherrscht, das in einem Dampferzeugungssystem zirkuliert, umfassend:

> einen Verteiler mit einer Durchflußzelle (28);
> ein Mittel (34) zum Ziehen einer Probe mit vorbestimmtem Volumen des zu untersuchenden Wassers ohne Luftkontakt und zum Zirkulieren der Probe durch den Verteiler;
> ein Mittel (24, 25) zum Injizieren eines vorbestimmten Volumens eines mit Eisen(II) reagierenden Reagens in die Probe, um in der Probe eine Farbintensität zu erzeugen, die zur Eisen(II)-Konzentration proportional ist;
> ein Mittel (26) zum Vermischen der Kombination aus Probe und Reagens im Verteiler;
> ein Mittel zum Steuern des Probenziehmittels, um den Fluß der Probe für eine erste Ruhephase anzuhalten, bevor das Gemisch aus Probe und Reagens die Durchflußzelle erreicht, um danach den Fluß der Probe wieder in Gang zu setzen, um anschließend den Fluß für eine zweite Ruhephase zu unterbrechen, nachdem das Gemisch aus Probe und Reagens die Durchflußzelle erreicht hat, und den Fluß dann wieder in Gang zu setzen, um den Verteiler durchzuspülen;
> ein Mittel (32, 36) zum Messen des Absorptionsvermögens der unvermischten Probe in der Durchflußzelle (28) während der ersten Ruhephase und zum Messen des Absorptionsvermögens der vermischten Probe in der Durchflußzelle (28) während der zweiten Ruhephase; und
> ein Mittel (40, 42) zum Umwandeln der gemessenen Absorptionswerte in Eisen(II)-Konzentrationswerte.

2. Vorrichtung nach Anspruch 1, weiters umfassend ein Mittel (40) zum Aussenden eines Spannungs- und TTL-Logiksignals entsprechend dem Wert der errechneten Eisen(II)-Konzentration.

3. Vorrichtung nach Anspruch 2, worin das Aussendemittel die Ausgangssignale konstant hält, bis die nächste Analyse diese aktualisiert.

4. Vorrichtung nach einem der vorangegangenen Ansprüche, worin das Steuermittel ein Mittel zum Anhalten des Probenflusses in einem Leerlauf enthält, um den nächsten Analysezyklus abzuwarten.

5. Vorrichtung nach einem der vorangegangenen Ansprüche, worin das Probenziehmittel eine Synchronpumpe (34) ist.

6. Vorrichtung nach Anspruch 5, weiters umfassend ein Mittel zum Bestimmen, ob die Pumpe mit der richtigen Frequenz arbeitet.

7. Vorrichtung nach einem der vorangegangenen Ansprüche, worin das Mischmittel eine Rohrwicklung (26) ist.

8. Vorrichtung nach einem der vorangegangenen Ansprüche, umfassend ein Mittel zum Umwandeln des Absorptionsvermögens zu gewissen Zeitpunkten in ein Spannungsanalog und zum Verwenden dieses Analogs zur Erzeugung einer Aufzeichnung der Eisen(II)-Konzentration über eine ähnliche Zeitspanne.

9. Vorrichtung nach einem der vorangegangenen Ansprüche, weiters umfassend Sensoren (22), um das Fließen einer Probe zur Zelle (28) und die Reagenszufuhr zu erfassen.

10. Vorrichtung nach einem der vorangegangenen Ansprüche, weiters umfassend einen Datenschreiber und Mittel als Schnittstelle zwischen dem Datenschreiber und dem Mittel zur Umwandlung der gemessenen Absorptionswerte in die Eisen(II)-Konzentration.

11. Vorrichtung nach einem der vorangegangenen Ansprüche, worin das Meßmittel eine Lichtquelle (32) mit geeigneter Wellenlänge und Intensität umfaßt.

12. Vorrichtung nach Anspruch 11, worin das Meßmittel weiters einen Detektor (36) zur Umwandlung der Lichtquellenintensität in Spannung umfaßt.

13. Vorrichtung nach Anspruch 12, worin die Durchflußzelle (28) durchsichtige Wände besitzt und die Lichtquelle (32) und der Detektor (36) angrenzend an die durchsichtigen Wände montiert sind, sodaß optische Messungen vorgenommen werden können.

14. Vorrichtung nach einem der vorangegangenen Ansprüche, worin das Steuermittel die erste Ruhephase etwa 18 Sekunden nach dem Kombinieren des Reagens mit der Probe durch das Einspritzmittel startet.

15. Vorrichtung nach Anspruch 14, worin das Steuermittel die zweite Ruhephase etwa 20 Sekunden nach der Messung während der ersten Ruhephase startet.

16. Vorrichtung nach Anspruch 15, worin das Meßmittel das Absorptionsvermögen der vermischten Probe etwa 15 Sekunden nach Beginn der zweiten Ruhephase abliest.

17. Vorrichtung nach Anspruch 16, worin das Steuermittel das Spülen der Vorrichtung für etwa 1 1/3 Minuten zuläßt.

18. Vorrichtung nach Anspruch 1, weiters umfassend eine Quelle (25) von Reagens, das mit Eisen(II) reagiert, um eine Farbintensität zu erzeugen, die proportional zur Eisen(II)-Konzentration ist;

wobei das Mittel (32, 36) zur Messung des Absorptionsvermögens der unvermischten Probe in der Durchflußzelle (28) während der ersten Ruhephase und zur Messung des Absorptionsvermögens der vermischten Probe in der Durchflußzelle (28) während der zweiten Ruhephase eine Lichtquelle und einen Photodetektor umfaßt;
und wobei das Mittel zur Umwandlung der gemessenen Absorptionswerte in die Eisen(II)-Konzentration ein Mittel zur Umwandlung des Absorptionswerts in ein zur Eisen(II)-Konzentration proportionales Spannungsanalog umfaßt.

19. Vorrichtung nach Anspruch 18, worin das Probenziehmittel folgendes umfaßt: eine mit einer Quelle des mit dem Reagens umzusetzenden Wassers verbundene Leitung;

ein Ventil mit einem mit der Leitung verbundenen Einlaß, einem weiteren mit der Reagensquelle verbundenen Einlaß und einem mit dem Mischmittel verbundenen Auslaß;
ein Mittel, um das Ventil zwischen einem Einlaß oder dem anderen Einlaß umzuschalten, sodaß entweder Wasser oder Reagens in das Mischmittel eintreten kann; und
eine Pumpe, um Wasser und Reagens zwangsweise in das Mischmittel und von dort in die Durchflußzelle (28) zu fördern.

20. Vorrichtung nach Anspruch 19, weiters umfassend Sensoren zum Nachweis der Gegenwart von Wasser in der Leitung oder von Reagens im Reagenseinlaß zum Ventil.

21. Vorrichtung nach Anspruch 18, worin das Mischmittel eine Rohrwicklung ist.

**EP 0 469 772 B1**

**22.** Vorrichtung nach Anspruch 18, worin das Umwandlungsmittel einen Analog-Digital-Wandler zur Umwandlung der Photodetektorspannung in ein digitales Signal, sowie einen Mikroprozessor umfasst, der programmiert ist, aus dem digitalen Signal die Eisen(II)-Konzentration zu errechnen.

**23.** Vorrichtung nach Anspruch 1, weiters umfassend eine Quelle eines Reagens, das mit Eisen(II) reagiert, um eine zur Eisen(II)-Konzentration proportionale Farbintensität zu erzeugen,

wobei das Probenziehmittel eine Leitung, ein Ventil zum Kombinieren eines vorbestimmten Volumens des Reagens aus einer Quelle mit der Probe und ein Mittel zum Öffnen und Schließen des Ventils umfaßt;
wobei das Mittel zur Steuerung des Probenziehmittels eine Pumpe umfaßt, um die mit Reagens kombinierte Probe zwangsweise in die Zelle zu fördern;
wobei die Vorrichtung weiters folgendes umfaßt: ein Timingmittel, um die Pumpe über eine vorbestimmte Zeitspanne auszuschalten, während das Absorptionsvermögen der Probe gemessen wird;
und ein Mittel zur Umwandlung des Absorptionswerts in ein zur Eisen(II)-Konzentration proportionales Spannungsanalog.

**Revendications**

**1.** Dispositif pour déterminer si de la corrosion, en termes de Fe(II) soluble, prévaut dans de l'eau circulant dans un système de production de vapeur, comprenant :

un collecteur comprenant une cellule d'écoulement (28) ;
un moyen (34) pour retirer un échantillon d'un volume d'eau prédéterminé à tester sans contact avec l'air et pour circuler l'échantillon à travers le collecteur ;
un moyen (24, 25) pour injecter dans l'échantillon un volume prédéterminé de réactant réactif avec Fe(II) pour produire une intensité de couleur dans l'échantillon proportionnelle à la concentration en Fe(II) ;
un moyen (26) pour mélanger l'échantillon et le réactif combinés dans collecteur ;
un moyen pour commander ledit moyen de retrait pour arrêter l'écoulement de l'échantillon pendant une première période de repos avant que l'échantillon et le réactant réactif mélangés atteignent la cellule d'écoulement, pour ensuite redémarrer l'écoulement de l'échantillon, pour ensuite arrêter l'écoulement pendant une seconde période de repos après que l'échantillon et le réactif mélangés aient atteint la cellule d'écoulement, et redémarrer ensuite l'écoulement pour balayer le collecteur ;
un moyen (32, 36) pour mesurer l'absorbance de l'échantillon non mélangé dans la cellule d'écoulement (28) pendant la première période de repos et pour mesurer l'absorbance de l'échantillon mélangé dans la cellule d'écoulement (28) pendant la seconde période de repos ; et
un moyen (40, 42) pour convertir les valeurs d'absorbance mesurées en concentration en Fe(II).

**2.** Dispositif selon la revendication 1 comprenant de plus un moyen (40) pour produire une tension et un signal logique TTL selon la valeur de la concentration calculée en Fe(II).

**3.** Dispositif selon la revendication 2 dans lequel le moyen de production précité maintient les sorties précitées au repos jusqu'à ce que l'analyse suivante les remette à jour.

**4.** Dispositif selon l'une quelconque des revendications précédentes dans lequel le moyen de contrôle comprend un moyen pour arrêter la circulation de l'échantillon dans un mode de repos pour attendre le cycle d'analyse suivant.

**5.** Dispositif selon l'une quelconque des revendications précédentes dans lequel le moyen de retrait précité est une pompe synchrone (34).

**6.** Dispositif selon la revendication 5 comprenant de plus un moyen pour déterminer si la pompe fonctionne à la fréquence correcte.

**7.** Dispositif selon l'une quelconque des revendications précédentes dans lequel le moyen mélangeur est une bobine de tubulure (26).

**8.** Dispositif selon l'une quelconque des revendications précédentes comprenant un moyen pour convertir de temps en temps l'absorbance en une tension analogique et utilisant cette tension analogique pour produire un enregistrement de concentration en matière ferreuse sur un intervalle de temps analogue.

9.  Dispositif selon l'une quelconque des revendications précédentes comprenant de plus des capteurs (22) pour détecter le passage d'un échantillon à la cellule (28) et détecter la fourniture de réactif.

10. Dispositif selon l'une quelconque des revendications précédentes comprenant de plus une centrale de mesure et un moyen pour interfacer la centrale de mesure avec le moyen précité de conversion des valeurs d'absorbance mesurées en concentration en Fe(II).

11. Dispositif selon l'une quelconque des revendications précédentes dans lequel le moyen de mesure précite comprend une source de lumière (32) de longueur d'onde et d'intensité convenables.

12. Dispositif selon la revendication 11 dans lequel le moyen de mesure comprend de plus un détecteur (36) pour transformer l'intensité de source de lumière en tension.

13. Dispositif selon la revendication 12 dans lequel la cellule d'écoulement (28) a des parois transparentes et la source de lumière (32) et le détecteur (36) sont montés adjacents auxdites parois transparentes de sorte que les mesures optiques peuvent être effectuées.

14. Dispositif selon l'une quelconque des revendications précédentes dans lequel le moyen de commande démarre la première période de repos à environ 18 secondes après que le moyen d'injection combine le réactif à l'échantillon.

15. Dispositif selon la revendication 14 dans lequel le moyen de commande démarre la seconde période de repos à environ 20 secondes après avoir prélevé la mesure pendant la première période de repos.

16. Dispositif selon la revendication 15 dans lequel le moyen de mesure lit l'absorbance de l'échantillon mélangé environ 15 secondes après que commence la seconde période de repos.

17. Dispositif selon la revendication 16 dans lequel le moyen de commande permet le balayage du dispositif pendant environ une et un tier de minutes.

18. Dispositif de la revendication 1 comprenant de plus une source (25) de réactant réactif avec Fe(II) pour produire une intensité de couleur proportionnelle à la concentration en Fe(II) ;

    le moyen précité (32, 36) pour mesurer l'adsorbance de l'échantillon non mélangé dans la cellule d'écoulement (28) pendant la première période de repos et pour mesurer l'absorbance de l'échantillon mélangé dans la cellule d'écoulement (28) pendant la seconde période de repos comprend une source de lumière et un photodétecteur ;
    et le moyen précité pour convertir les valeurs d'absorbance mesurées en concentration en Fe(II) comprend un moyen pour convertir la valeur d'absorbance en une tension analogique proportionnelle à la concentration en Fe(II).

19. Dispositif de la revendication 18 dans lequel le moyen de retrait précité comprend un conduit relié à une source d'eau à réagir avec le réactif ;

    une vanne ayant une entrée reliée au conduit, une autre entrée reliée à la source de réactif et une sortie reliée au moyen mélangeur ;
    un moyen pour commuter la vanne entre une entrée ou l'autre pour permettre à l'eau ou au réactif d'entrer le moyen mélangeur ; et
    une pompe pour forcer l'eau et le réactif dans le moyen mélangeur et de là dans la cellule d'écoulement (28).

20. Dispositif de la revendication 19 comprenant de plus des capteurs pour détecter la présence d'eau dans le conduit ou de réactif dand l'entrée de réactif à la vanne.

21. Dispositif de la revendication 18 dans lequel le moyen mélangeur est une bobine de tubulure.

22. Dispositif de la revendication 18 dans lequel le moyen de conversion comprend un convertisseur analogique-numérique pour convertir la tension du photodétecteur en un signal numérique, et un microprocesseur programmé pour calculer la concentration en Fe(II) à partir dudit signal numérique.

23. Dispositif de la revendication 1 comprenant de plus une source de réactant réactif avec Fe(II) pour produire une intensité de couleur proportionnelle à la concentration en Fe(II),

le moyen de retrait précité comprend un conduit, une vanne pour permettre à un volume prédéterminé de réactif d'une source d'être combiné à l'échantillon et un moyen pour ouvrir et fermer la vanne ;
le moyen précité pour commander ledit moyen de retrait comprenant une pompe pour forcer le réactif combiné à l'échantillon dans la cellule ;
ledit dispositif comprenant de plus un moyen de temporisation pour arrêter la pompe pendant une période prédéterminée pendant qu'on mesure l'échantillon pour l'absorbance ;
et un moyen pour convertir la valeur d'absorbance en une tension proportionnelle à la concentration en Fe(II).

fig.1.

fig.3.

TIME    Fe    DATE
16:49    5    10-5
17:9    196    10-5
19:9    187    10-5

22:9    182    10-5
23:59    180    10-5

0:59    183    10-6

Fig. 2.

fig.4.

SAMPLE AND REAGENT SENSOR ACTIVATION

VALVE PUMP

PUMP 82

SAMPLE 70

REAGENT SENSORS 72

DIGITAL I/O INTERFACE 58

STATUS LED'S 78A 78B 78C 78D

TTL OUT FOR HIGH Fe 80

RAM 54

EPROM 56

MICROPROCESSOR 52

RS232C COMMUNICATION 84

REAL TIME CLOCK 60

DATA LOGGER 50

12-BIT A/D CONVERTER 62

DETECTOR 36

28 32

12-BIT D/A CONVERTER 64

VOLTAGE OUT 42

EP 0 469 772 B1